# EUROPEAN PATENT APPLICATION

(11) **EP 2 700 429 A1**
(43) Date of publication of application: **26.02.2014**
(21) Application number: 12774428.2
(22) Date of filing: 13.04.2012
(51) Int. Cl.: A61M 35/00, A45D 34/04

(54) **DRUG SOLUTION APPLICATOR**

(30) Priority: 22.04.2011 JP 2011096035
(71) Applicant: R-Tech Ueno, Ltd., Tokyo 100-0011 (JP)
(72) Inventor: UNO, Tomoko, Tokyo 100-0011 (JP); HAYASHI, Tadashi, Tokyo 100-0011 (JP); HARADA, Yasuhiro, Tokyo 100-0011 (JP); TAJIMA, Masahiro, Tokyo 100-0011 (JP)
(74) Representative: Banzer, Hans-Jörg
(86) International application number: PCT/JP2012/060130
(87) International publication number: WO 2012/144437

(57) **Abstract**

The present invention provides an applicator, which holds an intended quantity of liquid medicament and applies the same to skin or mucous membrane without wasting. The applicator comprises a grip portion 10 for grasping; a neck portion 12, which can be elastically deflected, continuous from the grip portion 10; and a head portion 13, having a slit 14 for holding the liquid medicament supplied, continuous from the neck portion 12. When the head portion 13 is pressed against the skin or the mucous membrane, the liquid medicament is applied there while the neck portion 12 is elastically deflected.

## Description

### TECHNICAL FIELD

The present invention relates to an applicator for applying liquid medicament, which can be suitably used for applying liquid medicament to the skin around eyelashes, other skins, lip, mucous membranes in mouth, and so.

### BACKGROUND ART

The patent publication 1 discloses an applicator for applying liquid medicament of growing and fostering eyelashes. This applicator has a brush of resin bristles at the end of an elongated grip portion. The tip of the resin bristles is slantingly cut, thereby the contact area, when applying the liquid medicament to the skin around the eyelashes, is made larger. Thus, the applying efficiency is improved.

However, in this applicator, much liquid medicament goes into the bristles because of the capillarity, and much of the medicament in the bristles cannot be applied to the skin around eyelashes. Therefore, in order to apply much liquid medicament of a desired quantity, it is necessary to hold much quantity of the liquid medicament on the brush, and as a result, there arises a problem of wasting an expensive liquid medicament.

### PRIOR ART LITERATURE

### PATENT PUBLICATIONS

Patent Publication 1: PCT WO2010/065487

### SUMMARY OF THE INVENTION

### PROBLEM TO BE SOLVED BY THE INVENTION

The object of the present invention is to provide an applicator for applying liquid medicament, which is capable of holding a quantity of the liquid medicament and applying it to a target area without wasting.

### MEANS FOR SOLVING THE PROBLEMS

In order to achieve the above-mentioned object, an applicator for applying liquid medicament of the present invention comprises: a grip portion for grasping; a neck portion, which can be elastically deflected, continuous from the grip portion; and a head portion, having a slit for holding the liquid medicament supplied, continuous from the neck portion. Thus, when the head portion is pressed against a target area (skin around eyelashes, other skins, lip, mucous membranes in mouth, and so), the liquid medicament is applied to the target area while the neck portion is elastically deflected.

In the liquid medicament applicator of the present invention, when the head portion is pressed against the target area, the neck portion is elastically deflected. Thus, the stress (which would be induced because of the head portion pressed against the target area) can be eased, and the efficiency of applying liquid medicament can be improved. Further, an intended quantity of the liquid medicament can be surely held on the head portion having a slit by the surface tension. Besides, the liquid medicament supplied on the surface of the head portion can be applied to the target area without wasting. Note that efficiently applying an intended quantity of the liquid medicament to a particular target area, regarding a use of medicaments, is very important in order to follow the way and quantity of the medicament.

The applicator for applying liquid medicament of the present invention, it is preferable that thickness of the neck portion is made gradually thinner from the grip portion toward the head portion. With such the construction, it may be easy for a user to grasp the grip portion, while the tip side of the neck portion is made softer and thus the elastic deflection of the neck portion can be sure.

Further it is preferable that a rib, which prevents drop down of the liquid medicament, is provided at periphery of the head portion. With such the construction, an intended quantity of the liquid medicament can be surely held on the head portion.

### EFFECT OF THE INVENTION

In the liquid medicament applicator of the present invention, when the head portion is pressed against the target area, the neck portion is elastically deflected. Thus, the stress on the target area can be eased, and the efficiency of applying liquid medicament can be improved. Further, an intended quantity of the liquid medicament can be surely held on the head portion having a slit by the surface tension. Besides, the liquid medicament supplied on the surface of the head portion can be applied to the target area without wasting.

### BRIEF DESCRIPTION OF THE DRAWINGS

Fig. 1 shows a perspective view showing a practical use of a liquid medicament applicator according to the first embodiment of the present invention.
Fig. 2 shows the liquid medicament applicator according to the first embodiment, wherein (A) is a perspective view, (B) is a plan view, and (C) is a sectional view.
Fig. 3(A) and (B) are plan views showing modifications of the liquid medicament applicator of the first embodiment.
Fig. 4 shows a liquid medicament applicator according to the second embodiment, wherein (A) is a perspective view, (B) is a plan view, and (C) is a sectional view.
Fig. 5 shows a table, wherein the specifications of the portions of the liquid medicament applicator are described.
Fig. 6 shows a table, wherein the specifications of the embodiments of the liquid medicament applicator of the present invention, with which the experiments were conducted, are described.
Fig. 7 shows a table, wherein the results of the experiments are described.

### MODES FOR CARRYING OUT THE INVENTION

Hereinafter, embodiments of the invention are described with reference to the drawings.

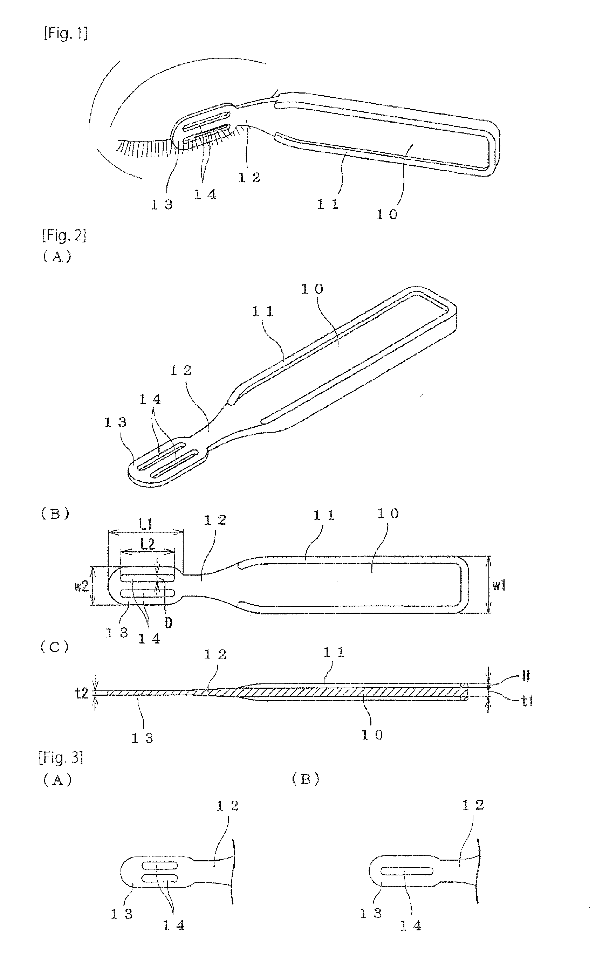

Figs. 1, 2 (A), 2(B), and 2 (C) show a liquid medicament applicator according to the first embodiment of the present invention. This liquid medicament applicator is for applying the liquid medicament of growing or fostering eyelashes to the skin around the eyelashes (a target area). This liquid medicament applicator, as shown in Fig. 2(A), is provided with a grip portion 10 for grasping, a neck portion 12 continuous to the grip portion 10, and a head portion 13 continuous to the neck portion 12. These portions are integrally made from resin material to form the liquid medicament applicator.

The grip portion 10 has a shape of an approximately rectangle, and a rib 11 is formed on the periphery of the grip portion 10. The thickness "t1" of the grip portion 10 and the height "H" of the rib 11, as shown in Fig. 2(C), are designed such that a user can easily grasp and the rigidity required for applying operation is obtained. Further, as shown in Fig. 2 (B), the lateral width "w1", perpendicular to the longitudinal direction, of the grip portion 10 is designed such that a user can easily grasp.

The neck portion 12, which is continuous to the tip of the grip portion 10, extends along the longitudinal direction of the grip portion 10. This neck portion 12 is formed to have a thickness, with which the neck portion 12 can be elastically deflected. Specifically, as shown in Fig. 2(C), the thickness of the neck portion 12 is tapered so as to become gradually thinner from the grip portion 10 to the head portion 13, and become more flexible at the tip side. The thickness of the neck portion 12 at the right end in the figure is the same as the thickness "t1" of the grip body, and the thickness of the neck portion 12 at the left end in the figure is the same as the thickness "t2" of the head portion 13. Further, as shown in Fig. 2(B), the neck portion 12 is tapered such that its lateral width becomes gradually narrower towards the head portion 13 at the tip. The overall length of the neck portion 12 is designed such that the head portion 13 can be sufficiently deflected with respect to the grip portion 10.

The head portion 13 is a spatula-like member, which extends along the longitudinal direction of the grip portion 10 to have a shape of approximately ellipse, and holds the liquid medicament in the slit 14 by the surface tension. One pair of slits 14 is provided, which extend along the longitudinal direction of the head portion 13. As shown in Fig. 2(C), the thickness "t2" of the head portion 13 is designed to be thinner than the thickness "t1" of the grip portion 10, and the head portion 13 can be elastically deflected. The width "D" of the slit 14, as shown in Fig. 2(B), is designed to be able to hold the liquid medicament by the surface tension, so as not to drop down. The lateral width "w2" of the head portion 13 is designed such that the slit 14 having the width "D" can be provided there, and there can be prevented from applying the liquid medicament to an area other than targeted. The overall length "L1" of the head portion 13 and the overall length "L2" of the slit 14 are determined depending on the intended quantity of the liquid medicament to be held.

When applying the liquid medicament of growing or fostering eyelashes to the skin around the eyelashes using this applicator, a user grasps the grip portion 10 in hand, and the liquid medicament held in a bottle or the like is dropped down onto the head portion 13. Then, as shown in Fig. 1, the head portion 13 is positioned above the eyelashes so as to extend horizontally, and the head portion 13 is pressed against the eyelid (skin) around the eyelashes. Then, the neck portion 12 will be elastically deflected and curved moderately. In this condition, the head portion 13 is forced onto the eyelid, but the stress on the skin is eased thanks to the elastic deformation of the neck portion 12.

Next, the grip portion 10 is made slide horizontally to move the head portion 13 along the eyelid. At this time, the eyelashes may pass through the slit 14, serving as a guide for the applying operation by the user. By such the sliding operation, the liquid medicament which is held on the surface of the head portion 13 having the slit 14 can be applied to the skin around the eyelashes, without wasting.

Thus, with the liquid medicament applicator of the present invention, the neck portion 12 can be deflected when the head portion 13 is pressed against the target area. Thus, the stress on the target area can be eased, and the efficiency of the applying operation can be improved. Further, an intended quantity of the liquid medicament can be surely held in the slit 14 of the head portion 13 by the surface tension. Besides, the liquid medicament held on the surface of the head portion 13 can be applied to the target area without wasting. Note that efficiently applying an intended quantity of the liquid medicament to a particular target area, regarding a use of medicaments, is very important in order to follow the way and quantity of the medicament, and especially preferred.

Note that, regarding the head portion 13 of the liquid medicament applicator of the first embodiment, the lateral width "w2", and the overall lengths "L2" and the number of the slit 14 can be variously changed. Also in such the case, the same function and effect can be obtained. For example, as shown in Fig. 3 (A), the lateral width "w2" of the head portion 13 can be made narrower, and the overall length "L2" of the slit 14 can be made shorter. Further, as shown in Fig. 3(B), the lateral width "w2" of the head portion 13 can be narrower, and only one slit 14 can be provided in the center of the head portion 13.

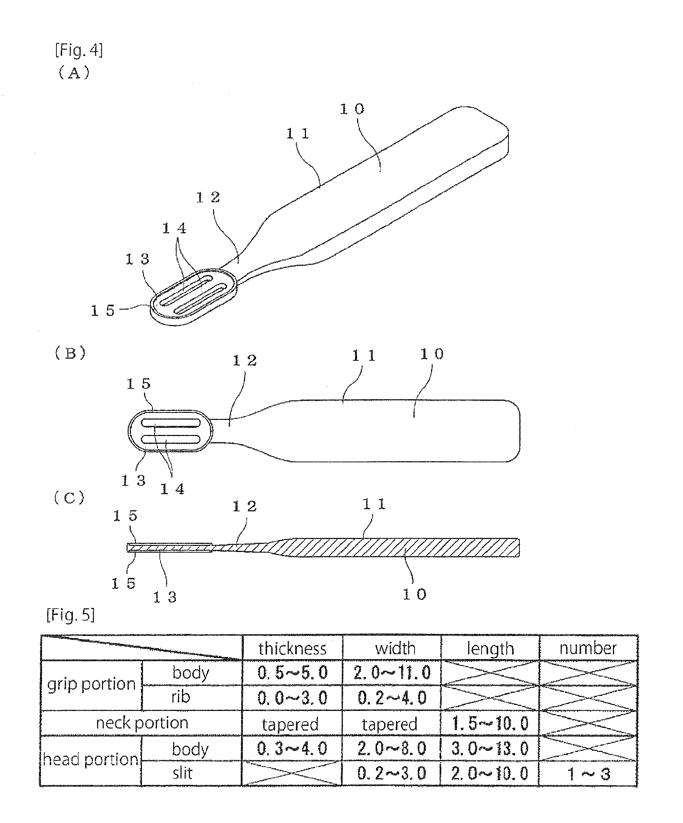

Figs. 4(A), 4(B) and 4(C) show a liquid medicament applicator of the second embodiment. In this second embodiment, thickness "t1" of the grip portion 10 is made thicker, and the rib 11 is omitted. Further, at periphery of the head portion 13, a rib 15 which prevents the drop down of the liquid medicament is formed. With this construction, the same function and effect as the first embodiment can be obtained, and besides, the drop down of the liquid medicament held on the head portion 13 can be surely prevented. Thus, an intended quantity of the liquid medicament can be surely held on the head portion 13, and the liquid medicament can be applied to a particular target area.

Note that the number and the size of the grip portion 10, the neck portion 12, and the head portion 13 of each embodiment can be appropriately changed, as exemplified in Fig. 5. In any case, it is possible to apply the liquid medicament, while the head portion 13 is pressed against a target area to elastically deflect the neck portion 12.

Regarding the thickness "t1" of the grip portion 10, in case of too thinner or too thicker, a user may feel difficult to grasp it. Thus, the thickness "t1" is preferably thicker than 0.5 mm, and thinner than 5.0 mm. Further, regarding the lateral width "w1" of the grip portion 10, in case of too narrower or too wider, a user may feel difficult to grasp it. Thus, the lateral width "w1" is preferably wider than 2.0 mm, and narrower than 11.0 mm.

If sufficient rigidity is obtained with the thickness "t1" of the grip portion 10, it is not necessary to provide the rib 11. In case the rigidity is insufficient, it is necessary to provide the rib 11. However, regarding the height "H" (thickness) of the rib 11, in case of too higher, a user may feel difficult to grasp it because of tall bump. Thus, the height "H" is preferably higher than 0.0 mm, and shorter than 3.0 mm. Regarding the lateral width of the rib 11, in case of too narrower, sufficient rigidity will not be obtained. On the other hand, in case of too wider, the proportion of the rib 11 in the whole area of the grip portion 10 become large, and thus a user may hardly recognizes the rib 11 when grasping, which may spoil the merit of easy-grasping with fingers being caught. Thus, the lateral width of the rib 11 is preferably wider than 0.2 mm, and narrower than 4.0 mm.

The neck portion 12 needs to be softer at the tip-side for making the head portion 13 flexibly curved along with the skin when applying the liquid medicament. It is necessary to make the grip portion 10, the neck portion 12, and the head portion 13 what they can be visually distinguished, for a user to easily recognize how to use (what portion is to be grasped, what portion is to be pressed against the target area with the liquid medicament, and so) . Thus, the neck portion 12 is preferably formed to have a shape of narrow and tapered. The overall length of the neck portion 12 is preferably longer than 1.5 mm, and shorter than 10.0 mm.

Regarding the thickness "t2" of the head portion 13, in case of too thinner, molding will become difficult. On the other hand, in case of too thicker, elastic deformation will become hard such that the stress on the target area will be increased when pressing onto the target area. Thus, the thickness "t2" of the head portion 13 is preferably thicker than 0.3 mm, and thinner than 4.0 mm. Regarding the lateral width "w2" of the head portion 13, in case of too narrower, an intended number of the slit 14 cannot be provided there. On the other hand, in case of too wider, the liquid medicament would be excessively applied beyond the target area. Thus, the lateral width "w2" of the head portion 13 is preferably wider than 2.0 mm, and narrower than 8.0 mm. Regarding the overall length "L1" of the head portion 13, in case of too shorter, the slit 14 with sufficient overall length "L2" cannot be provided there. On the other hand, in case of too longer, the head portion 13 would not fit to the target area and make it difficult to apply the liquid medicament. Thus, the overall length "L1" of the head portion 13 is preferably longer than 3.0 mm, and shorter than 13.0 mm.

Regarding the width "D" of the slit 14, in case of too narrower, sufficient quantity of the liquid medicament would not be held by the surface tension. On the other hand, in case of too wider, the liquid medicament would drop down. Thus, the width "D" of the slit 14 is preferably wider than 0.2 mm, and narrower than 3.0 mm. Regarding the overall length "L2" of the slit 14, in case of too shorter, sufficient quantity of the liquid medicament would not be held. On the other hand, in case of too longer, the overall length "L1" of the head portion 13 will also become too long. Thus, the overall length "L2" of the slit 14 is preferably longer than 2.0 mm, and shorter than 10.0 mm. Regarding the number of the slit 14, in case of too fewer, sufficient quantity of the liquid medicament would not be held. On the other hand, in case of too many, the lateral width "w2" of the head portion 13 will become wider. Thus, the number of the slit 14 is preferably 1 to 3.

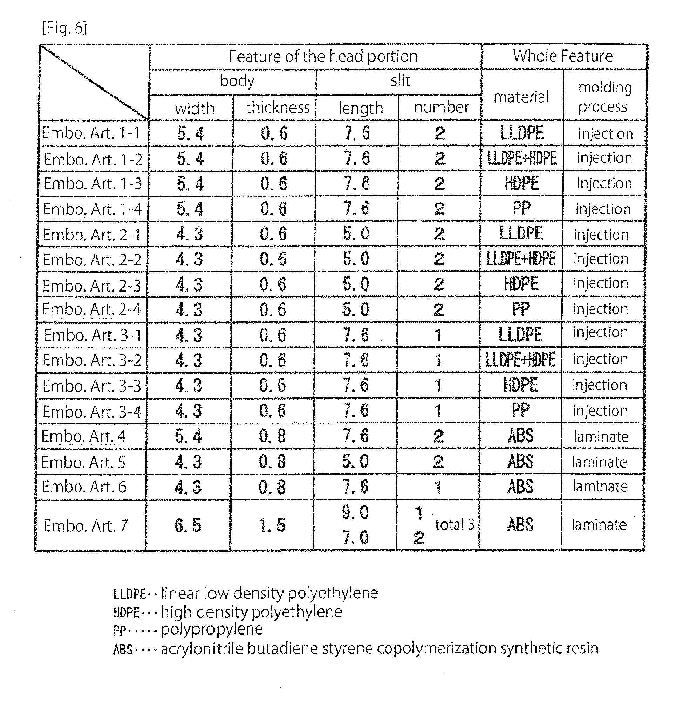

In order to confirm the effect of the liquid medicament applicator of the present invention, as shown in Fig. 6, the inventors of this application made some several samples (embodiments of the invention) with various size of the head portion 13, the number of slit 14, the materials, and the way of manufacturing. And the experiments described below were conducted.

Specifically, in the embodiment article 1, the head portion 13 has a lateral width "w2" of 5.4 mm and a thickness "t2" of 0.6 mm, and there are provided two slits 14 having an overall length "L2" of 7.6 mm and a width "D" of 1.0 mm. The sizes of other embodiment articles 2 to 7 are as shown in Fig. 6. Note that the embodiment articles 1-1 to 1-4 are different only in that they are made from the different materials of resin. It is also true among the embodiment articles 2-1 to 2-4, and also among the embodiment articles 3-1 to 3-4. The embodiment articles 1 to 3 were made by the injection molding, wherein melted resin is injected into a metallic mold. The embodiment articles 4-7 were made by the laminate molding, wherein the resin is applied in layers.

Besides, as a comparative article 1, the applicator disclosed in the Patent Publication 1 was provided, which has a brush of many resin bristles. As a comparative article 2, there was provided a stick, something like a cotton applicator, which has a head portion where many fibers are implanted. As a comparative article 3, there was provided a stick, which is like the comparative article 2, but has lower density of fibers implanted than that in the comparative article 2.

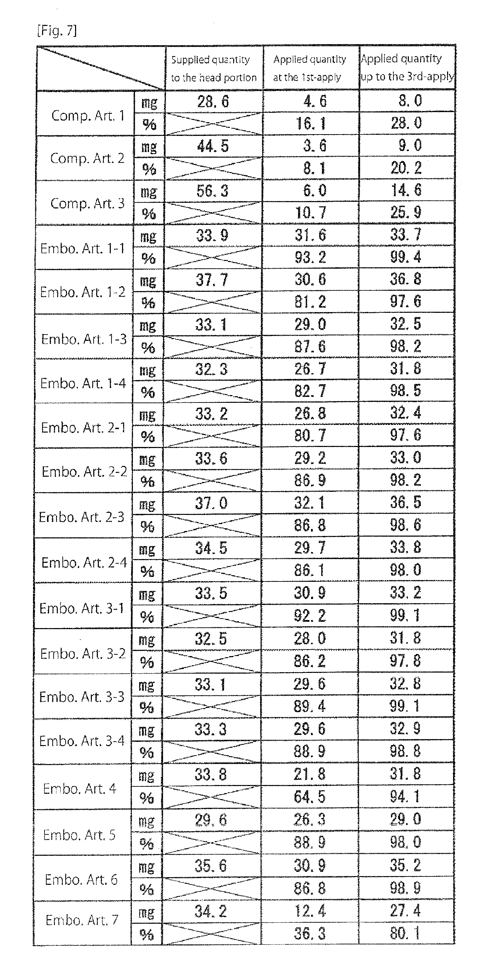

The experiments using these comparative articles 1 to 3 and the embodiment articles 1-7 were as follows. Liquid medicament was supplied to the head portion 13, and the quantity of the supplied liquid medicament was determined. Next, the liquid medicament held on the head portion 13 was applied to the skin around eyelashes, and the quantity of the applied medicament at this 1st-apply was determined. Based on the quantity of the supplied liquid medicament and the quantity of the 1st-apply, the apply-rate (%) after the 1st-apply was checked. After that, without supplying further liquid medicament to the head portion 13, a 2nd-apply and a 3rd-apply were conducted, and the total quantity of the applied medicament up to the 3rd-apply was determined. Based on the quantity of the supplied liquid medicament and the quantity up to the 3rd-apply, the apply-rate (%) after the 3rd-apply was checked. The results of the experiments are shown in Fig. 7.

In the comparative article 1, the quantity of the supplied liquid medicament was 28.6 mg, and the quantity of the 1st-apply to the skin was 4.6 mg. This means that 16.0% of the supplied liquid medicament 28.6 mg was applied to the skin. After the 3rd-apply, totally, the quantity of the applied medicament was 8.0 mg, which means that 27.9% of the supplied liquid medicament 28.6 mg was applied to the skin. Incidentally, in this comparative article 1, some excessive liquid medicament flew down the skin when applying the liquid medicament, the quantity of which was 2.5 mg. This means that 8.7% of the supplied liquid medicament 28.6 mg was wasted. The results of other comparative articles 2 and 3 are as shown in Fig. 7.

From the results, it can be understood that more than about 72% of the liquid medicament supplied is wasted, in the conventional applicators (comparative articles 1 to 3).

On the other hand, in the embodiment article 1-1 of the present invention, the quantity of the supplied liquid medicament was 33.9 mg, and the quantity of the 1st-apply to the skin was 31.6 mg. This means that 93.2% of the supplied liquid medicament 33.9 mg was applied to the skin. After the 3rd-apply, totally, the quantity of the applied medicament was 33.7 mg, which means that 99.4% of the supplied liquid medicament 33.9 mg was applied to the skin. The results of other embodiment articles 1-2 to 7 are as shown in Fig. 7.

From the results, it can be understood that in the embodiment articles 1 to 7 of the present invention, after the 3rd-apply, the apply-rate (%) was all more than 80%. This value does not vary so much with the difference of the shape and the material of the head portion 13 of the applicator. But note, in the embodiment articles 4 to 7 made by the laminate molding, it is found that the more the number and the overall length of the slit 14 become, the lower the apply-rate becomes. The reason of which is supposed that in the case of the laminate molding there may be little crevices between the layers, and the liquid medicament may go therein. Thus, the liquid medicament applicator may be preferably made by injection molding. However, even when made by laminate molding, the apply-rate was significantly improved compared with the comparative articles 1 to 3. Thus, an applicator of the present invention made by the laminate molding is not bad.

Considering from these results, the liquid medicament applicator of the present invention can be manufactured using the common process and the material in this field.

Process for manufacturing the liquid medicament applicator of the present invention are, for example, casting, compression molding, transfer molding, injection molding, extrusion molding, inflation molding, calendaring, blow molding, vacuum forming, laminate molding, spray up molding, and foaming.

As plastic materials there can be used, for example, high density polyethylene (HDPE), medium density polyethylene (MDPE), low density polyethylene (LDPE), linear low density polyethylene (LLDPE), polypropylene (PP), polyvinyl chloride (PVC), polyvinylidene chloride, polystyrene (PS), polyvinyl acetate (PVAc), polytetrafluoroethylene (PTFE), ABS resin (acrylonitrile butadiene styrene resin), AS resin, acrylic resin (PMMA,), polyamide (PA), polyacetal (PCM), polycarbonate (PC), modified polyphenylene ether (m-PPE, modified PPE and PPO), polybutylene terephthalate (PBT), polyethylene terephthalate (PET), fiberglass reinforced polyethylene terephthalate (GF-PET), cyclicpolyolefin (COP), polyphenylene sulfide (PPS), polytetrafluoroethylene (PTFE), polysulfone (PSF), polyethersulfone (PES), amorphous polyalylate (PAR), liquid crystal polymer (LCP), polyether ether ketone (PEEK), thermoplastic polyimide (PI), and polyamide imide (PAI).

Note that the practical uses of the liquid medicament applicator of the present invention is not limited to the use for eyelashes, although the described embodiment was directed to where the liquid medicament of growing and fostering eyelashes was applied to the eyelid around the eyelashes. For example, the liquid medicament applicator of the present invention can be used for applying liquid medicament to stomatitis, other than applying liquid medicament to the skin around eyelashes. That is, the liquid medicament applicator of the present invention can be used for applying liquid medicament to various skins, lip, and mucous membranes in mouth.

Further, the liquid medicament applicator of the present invention can also be used for applying gelatinous liquid medicament of increased viscosity to the skin and the mucous membrane. In the second embodiment of the liquid medicament applicator, thanks to the rib 15 which would prevent the drop down of the liquid medicament, the applicator can also be used preferably for applying liquid medicament flowable enough to drop down (solution having viscosity of about 500 cP). Liquid medicament of water solubility or oiliness can also be preferably used.

### EXPLANATION OF REFERENCE NUMERALS

10: Grip portion
11: Rib
12: Neck portion
13: Head portion
14: Slit
15: Rib

## Claims

1. An applicator for applying liquid medicament, comprising:
a grip portion for grasping,
a neck portion, which can be elastically deflected, continuous from the grip portion, and
a head portion, having a slit for holding the liquid medicament supplied, continuous from the neck portion,
wherein when the head portion is pressed against a target area, the liquid medicament is applied to the target area while the neck portion is elastically deflected.

2. The applicator for applying liquid medicament according to claim 1, wherein thickness of the neck portion is made gradually thinner from the grip portion toward the head portion.

3. The applicator for applying liquid medicament according to claim 1 or 2, wherein a rib, which prevents drop down of the liquid medicament, is provided at periphery of the head portion.
